# EUROPEAN PATENT APPLICATION

(11) **EP 2 733 214 A1**
(43) Date of publication of application: **21.05.2014**
(21) Application number: 13191117.4
(22) Date of filing: 31.10.2013
(51) Int. Cl.: C12P 7/10, C12N 1/16, C12N 1/22, C12R 1/85, A23K 1/00

(54) **Eliminating the need of acidification in bioethanol production**

(30) Priority: 15.11.2012 US 201261726785 P
(71) Applicant: ANITOX CORPORATION, Lawrenceville, GA 30043 (US)
(72) Inventor: Pimentel, Julio L., Buford, GA Georgia 30519 (US); Richardson, Kurt, Maysville, GA Georgia 30558 (US)
(74) Representative: Chung, Hsu Min

(57) **Abstract**

An improved ethanol fermentation process with decreased use of acidifiers by adding a composition containing an aldehyde, a fatty acid, a terpene and a surfactant. The method comprising:
a) mixing a fermentation feedstock with a fermentation broth containing yeast and/or an enzyme,
b) treating said mixture by adding a composition to the fermentor containing:
10 - 90 wt. % of an antimicrobial aldehyde, preferably selected from the group consisting of formaldehyde, para-formaldehyde, glutaraldehyde, and mixtures thereof,
1- 50 wt. % of a surfactant having an HLB from 4 to 18,
0 - 20 wt. % of an antimicrobial terpene, or essential oils,
1- 50 wt. % of organic acids selected from C₁ to C₂₄ fatty acids, their salts, glycerides and esters thereof, and
1- 50 wt. % water;
wherein the concentration of aldehyde in the fermentor is from about 0.25 to 3 kg/MT of fermentation feedstock, and

c) isolating ethanol and improving yield.

## Description

### BACKGROUND OF THE INVENTION

### FIELD OF THE INVENTION

An improvement of the ethanol fermentation process by decreasing the use of acidifiers with the addition of a composition containing an aldehyde, a fatty acid, a terpene and a surfactant.

### BACKGROUND

Ethanol, a biofuel from renewable resources, is produced from the starch of cereal grains (corn, sorghum, wheat, triticale, rye, malted barley, rice), tuber crops (potatoes) or by direct use of the sugar in molasses, sugarcane juice, sugar beet juice, by fermentation of cellulose-based material (switchgrass, pine trees).

Ethanol production through anaerobic fermentation of a carbon source by the yeast *Saccharomyces cerevisiae* is one of the best known biotechnological processes and accounts for more than 35 billion liters of ethanol per year worldwide (Bayrock, 2007).

Ethanol production from cereal grains begins with the hydrolysis of starch resulting in the conversion of amylose, a mostly linear α-D-(1-4)-glucan, and branched amylopectin, a α -D-(1-4)-glucan which has α -D-(1-6) linkages at the branch point, into fermentable sugars which are subsequently converted to ethanol by yeast (Majovic, 2006) or bacteria (Dien, 2003). Bacteria can convert cellulose-containing material into fermentable sugars for the production of ethanol; these include *Zymomonas spp.,* engineered *E. coli, Klebsiella oxytoca, Zymomonas mobilis, Acetivibrio celluloyticus* and others (Dien, 2003). In the case of sugarcane and sugar beet, yeast directly utilizes the sugar to convert it to ethanol.

In the production of ethanol from grains, even though the optimal pH for yeast growth is above 5.0 (van den Bersselaar and Oosting, 2010; Joostte and Peeters, 2010), a low pH of the fermentable solution is used in order to decrease the number of bacteria. The pH of the solution is decreased from a pH of 5.0-6.0 down to a pH of 4.2-4.6. Sulfuric acid is commonly used to reduce the pH. The negative impact of acidification is the accumulation of sulfur in the resulting wet distillers grain and dry distillers grains plus solubles (WDG and DDGS, respectively).

High levels of sulfur (S) in animal feed decreases body weight gain and can produce polioencephalomalacia (PEM). Polioencephalomalacia is a neurological disorder characterized by necrosis of the cerebral cortex. Clinical symptoms of PEM include increased respiration rates, progressive blindness, depressed feed intake, head pressing, and the animal's breath has a smell of rotten eggs (Solange, 2011). The concentration of sulfur (S) in DDGS has been reported from 0.33 to 0.74% dry matter (DM), and 0.36 - 0.60% for wet distillers grains plus solubles (WDGS). Condensed distillers solubles may contain from 0.8 to 1% S on a dry matter basis. In a study, 9 out of 50 animals fed dry-rolled corn finishing diets containing 50% DDGS (0.6% S) were diagnosed with polioencephalomalacia (PEM), and some the animals died (Solange, 2011). In similar work, Vanness et. al. (2009) found that WDGS averaged 0.79% S (DM basis) in 1,200 samples from six Nebraska ethanol plants. If WDGS are fed at high levels in finishing diets, the dietary S levels may exceed nutritional guidelines. The National Research Council (1996) suggests the limit for S in diets should not exceed 0.40%. These data suggest that diets at or below 0.46% S have a low risk of producing PEM. Vanness et. al. (2009) suggested the use of phosphoric acid instead of sulfuric acid in ethanol fermentation can result in the production of low sulfur WDG and DDGS. The substitution of phosphoric acid for sulfuric acid did not affect fermentation or ethanol yields. Since phosphoric acid does not disassociate as readily as sulfuric acid, approximately 2.5 times more phosphoric acid is required to provide the same pH control increasing cost. Rasmussen (2011) using ozonation in a laboratory-scale at dosages from 26-188 mg/L in combination with low sulfuric acid to adjust the pH prior to ethanol fermentation resulted in lower levels of lactic and acetic acid, indicating less bacterial activity. The lower ozone dosages in the range applied achieved higher ethanol yields.

Acidification is also used in the production of ethanol from sugarcane, the pH of the yeast solution is decreased to a pH of 2.5- 3.0 with sulfuric acid in order to decrease yeast flocculation and reduce bacteria load since the yeast has been recycled from a previous continuous batch fermentation. Viegas (2011) suggested the used of propolis extract to decrease the use of antimicrobials thus eliminating the need to decrease pH. The elimination of antimicrobials in the sugarcane industry is of great concern since some percentage of recycled yeast is used for animal and human consumption and antimicrobial residues can result in antimicrobial-resistant bacteria.

Patent Application US2012/0009639 suggests a composition of poly(hexamethyl biguanide), an antibiotic agent, and a surfactant agent, to prevent the undesired microbial growth during ethanol fermentation. The amount of sulfuric acid used in the treatment of yeast can be reduced due to the reduced number of bacteria. Patent Application US2009/0215127 suggests a pH adjustment free system by the addition of a combination of phytase and amylase during the liquefaction of starch prior to fermentation to produce ethanol. Patent Application US2010/0297719 and Patent Application US2011/0027846 provide a formulation of an organic biocide, a quaternary ammonium compound, a formaldehyde-releasing compound and a guanidine-based compound to decrease microbial contamination during sugar fermentation. The mentioned US patent applications do not provide data for ethanol yield.

The present invention can be used in any type of ethanol production plant. The examples includes: dry milling, wet milling, dry grinding and sugarcane ethanol. In the dry milling process, the entire corn (Zea mays) kernel or other starchy material is ground into flour and mixed with water to form a slurry. The mixture is then steam cooked to gelatinize the starch and decrease bacterial contamination. This mixture when cooled is transferred to fermenters where yeast and enzymes are added to convert the fermentable sugars to ethanol. After fermentation, the resulting mixture is transferred to distillation columns where the ethanol is separated. The solids remaining after fermentation and ethanol separation are processed into wet and dried distiller grains with solubles (WDG and DDGS), which is used for animal production, e.g. poultry, swine, and cattle feed (RFA, 2006). In wet milling the grain is soaked or steeped in water to facilitate separation of the grain into its basic nutritional components, such as corn germ, fiber, gluten and starch. After steeping, the corn slurry is processed through a series of grinders and the components are separated. The gluten component is filtered and dried to produce corn gluten meal (CGM), a high-protein product used as a feed ingredient in animal operations. The starch and any remaining water from the mash are then processed in one of three ways: fermented into ethanol, dried and sold as dried or modified corn starch, or processed into corn syrup (RFA, 2006). In the dry grinding process, the whole grain is ground and fermented to ethanol bypassing conventional starch gelatinization (cooking) conditions (Thomas, 2001). In sugarcane ethanol production, ethanol is produced from the juice after pressing acid-washed cane stalks. After sugarcane juice is extracted it is transformed into alcohol through a fermentation process using yeast. Sugar from sugarcane is readily available to yeast so fermentation requires only between 4 to 12 hours, compared to 72 hours for fermentation using cereal grains.

The main objective of acidification of starchy or sugary slurries is to control bacterial growth, prevent yeast flocculation and decrease foaming of the slurry. A variety of gram positive and gram negative bacteria have been isolated from fuel ethanol fermentation including species of *Lactobacillus, Pediococcus, Staphylococcus, Enterococcus, Acetobacter, Gluconobacter, A.pasterurianus, B. Subtilis, Leuconostoc mesenteroides, Weissella paramesenteroides* and *Clostridium* (Bischoff, 2009). Almost two thirds of the bacteria isolated were species of lactic acid bacteria, e.g. *Lactobacillus* (Skinner, 2007). In sugarcane ethanol production, *Leuconostoc* has been reported to negatively influence ethanol yield. The contamination of carbohydrate slurry or sugary slurry during the course of alcoholic fermentation results in a) decreased ethanol yield, b) increased channeling of carbohydrates for the production lactic acid and other small chain fatty acids, c) a rapid loss of the yeast viability after exhaustion of fermentable sugars, and d) decreased proliferation of yeast in the mash in which the contaminating Lactobacilli has already grown to a high number (Thomas, 2001). In a continuous fermentation, yeast is recycled and added back to the fermenter. This recycled filtrate makes up 10-12% of the total batch volume. In the continuous fermentation plant, two different microbial problems exist. The first problem is a bacterial contamination issue. When yeasts are recycled, any bacterial contaminant in the filtrate is also recycled. A second source of bacterial contamination is through the production of biofilms in the fermenters and transport lines. In the batch fermenter, recycled yeasts are not used. Bacterial contamination is less severe and enters the system through the raw sugar juice/molasses or from biofilms in the process equipment.

Currently, monensin and virginiamycin are used in bioethanol plants (Bischoff, 2007). The recommended dose of virginiamycin in fuel ethanol fermentations is generally 0.25 to 2.0 ppm (Bischoff, 2009) but the Minimum Inhibitory Concentration (MIC) varies from 0.5 to greater than 64 ppm (Hynes, 1997). Virginiamycin, penicillin, erythromycin, kanamycin, tetracycline, ampicillin, streptomycin, monensin and nalidixic acid have been used during sugarcane ethanol production. Antibiotic resistance has been found to penicillin and ampicillin (97% of bacteria are resistant) and to virginiamycin (48% of the bacteria are resistant). The maximum recommended usage rate is 20 mg/L. The use of 10 ppm Kamoran (trade name of monensin) or a mixture of penicillin 10 ppm and tetracycline have been used to prevent sugarcane deterioration (Payot, 2004).

Most bacteria, with the exception of *Lactobacillus casei,* can be controlled by hydrogen peroxide at concentrations of 1 to 10 mM in the ethanol fermentation process (Narendranath, 2000). Urea hydrogen peroxide (UHP) exhibits excellent bactericidal activity against Lactobacillus and also has an important advantage of providing usable nitrogen in the form of urea for stimulating yeast growth and fermentation rates (Narendranath, 2000). Sulfites demonstrate bactericidal activity only in the presence of oxygen and were more effective in killing facultative *L. casei* which possess high levels of hydrogen peroxide related enzymes, including peroxidase (Chang, 1997). Succinic acid at levels of 600 mg/L reduces Lactobacillus levels by 78%, in the presence of ethanol the reduction is up to 96% (Oliva-Neto 2004). A microbial adherence inhibitor in the form of fowl egg antibodies and specific to lactic acid-producing microorganisms has been developed for use in fermenters (Nash 2009). Chlorine dioxide is recommended from 40 to 750 ppm but causes corrosion in the stainless steel fermentation tanks. Beta acids have antimicrobial properties but its use still in its infancy. BioZyn is claimed to be a "natural product" with unknown composition, but it is new in the market. Ozone and electromagnetic radiation are also new alternatives. A combination of 8.6 ppm nisin and 0.1% Tween 20 can be used to delay the lag phase of Lactobacillus for up to 12 hours (Franchi et.al., 2006). The use of nisin and EDTA decreased in vitro growth of *Lactobacillus casei* (Limayen, et. al 2011). The mixture of peracetic acid and hydrogen peroxide has also been used to control microorganisms in ethanol fermentation (DeLasan MP; a product from DeLaval, 2011).

Despite efforts to prevent contamination through cleaning and disinfecting saccharification tanks and continuous yeast propagation systems, biofilms can act as reservoirs of bacteria that continuously reintroduce contaminants (Bischoff, 2009). Biofilm cells are organized into structured communities enclosed in a matrix of extracellular material. They are phenotypically different from planktonic or suspended cells (Berit et. al. 2002). Damaged lines or pipes that are abraded or scratched create surfaces where organisms can more easily attach (Perez-Conesa, et.al. 2006).

Even though many products are commercially available for bacteria control, acidification is still being used. Using the product of this invention, acidification is not required. Various patents and publications are referenced throughout this specification. The disclosures of each document are hereby incorporated by reference in their entirety.

### REFERENCES

Bayrock, D., 2007. Method of reducing the growth of lactobacillus in a process of ethanol production by yeast fermentation comprising adding a pristinamycin type antimicrobial agent and/or a polyether ionophore antimicrobial agent dissolved in an organic solvent. PCT patent # WO 2007/145858.
Berit, A. G.S. Baillie and L.J. Douglas, 2002. Mixed species biofilms of Candida albicans and Staphylococcus epidermis. J. Med Microbiol 51: 344-349.
Bischoff, K.M., S. Liu, T.D. Leathers and R.E. Worthington, 2009. Modeling bacterial Contamination of Fuel Ethanol Fermentation. Biotechno. Bioeng. 103: 117-122.
Bischoff, K.M., K.A. Skinner-Nemec and T.D. Leathers, 2007. Antimicrobial susceptibility of Lactobacillus species isolated from commercial ethanol plants. J. Ind. Microbiol. Biotechnol. 27: 39-45.
Chang I.N., B.H. Kim and P.K. Shin, 1997. Use of sulfite and hydrogen peroxide to control bacterial contamination in ethanol fermentation. Applied and Environmental Microbiology 63(1): 1-6.
DeLasan MP a product from DeLaval. 2011.
Dien, B.S., M.A. Cotta and T.W. Jeffries, 2003. Bacteria engineered for fuel ethanol production: current status. Appl. Microbiol. Biotechnol. 63: 258-266.
Franchi, M.A., G.E. Serra and M. Cristianini, 2006. The use of biopreservatives in the control of bacterial contaminants of sugarcane alcohol fermentation 68(7):2310-2315.
Hynes, S.H., Kjarsgaard, K.C. Thomas and W.M. Ingledew, 1997 . Use of virginiamycin to control the growth of lactic acid bacteria during alcohol fermentation. J. Industrial Microbiology and Biotechnology 18: 284-291.
Joosten, M. and M. Peeters, 2010. Yeast and fermentation: the optimal pH level. From Philips van Horne sg. Weert, The Netherlands. June 2nd 2010.
Limayen A., I.B, Hanning, A. Muthaiyan, K. Illeghems, J.W. Kim, P.G. Crandall, C.A. O'bryan and S.C. Ricke, 2011. Alternative antimicrobial compounds to control potential Lactobacillus contamination in bioethanol fermentation. J. Environ Sci Health 46(8):709-714.
Majovic, L, S. Nikolic, M. Rakin and M. Vukasinovic, 2006. Production of Bioethanol from Corn Meal Hydrolyzates. Fuel 85: 1750-1755.
Narendranath, N.V., K.C. Thomas and W.M. Ingledew, 2000. Urea hydrogen peroxide reduces the number of lactobacilli, nourish yeast, and leaves no residues in the ethanol fermentation. Applied and Environmental Microbiology 66(10): 4187-4192.
Nash, P., 2009. Immunogen adherence inhibitor directed to lactobacillus organisms and method of making and using it. United States Patent Application #20090117129
Oliva Neto, P., M.A. Ferreira and F. Yokoya, 2004. Screening for yeast with antibacterial properties from ethanol distillery. Bioresource Technology 92: 1-6.
Payot, T. 2004. Kamoran using in sugar beet production to improve the quality of diffusion step UNGDA, www.ungda.com.
Perez-Conesa, D., L Mclansboough and J. Weiss, 2006. Inhibition and inactivation of Listeria monocytogenes and Escherichia coli O157:H7 colony biofilms by micellar-encapsulated eugenol and carvacrol. J. Food Protection 69(12): 2947-2954.
Rasmussen, 2011. "Enhancing dry-grinding corn ethanol production with fungal cultivation anf ozonation. PhD. dissertation Thesis Iowa State University.
RFA "Renewable Fuels Association" 2006 and 2009.
Skinner-Nemec, K.A., N. N. Nichols and T.D. Leathers, 2007. Biofilm formation by bacterial contaminants of fuel ethanol production. Biotechnol. Lett. 29: 379-383.
Skinner, K.A. and T.D. Leathers, 2004. Bacterial Contaminants of Fuel Ethanol Production. J. Ind. Microbiol. Biotech. 31: 401-408.
Solange U., 2011. "Utilization of Distillers Grains in Feedlot Cattle Diets" PhD. Dissertation, Department of Animal Sciences and Industry Kansa State University, Manhattan, Kansas.
Thomas, K.C., S.H. Hynes and W.M. Ingledew, 2001. Effect of lactobacilli on yeast growth, viability and batch and semi-continuous alcoholic fermentation on corn mash. J. Applied Microbiology 90: 819-828.
van den Bersselaar, E. and D. Oosting, 2010. Alcoholic fermentation: the optimal pH. From Ds. Pierson College, 's-Hertogenbosch, The Netherlands.
Vanness, S., J. Terry, G. Klopfenstein and E. Erickson, 2009. Sulfur in Distillers Grains, Nebraska Beef Cattle Reports. University of Nebraska - Lincoln.
Viegas, E., 2011. Bacterial properties of green propolis against bacterial contaminants on the ethanol fermentation. Master Dissertation, Luiz de Queioz College of Griculture, Brazil.

### SUMMARY OF THE INVENTION

An object of the invention is to provide a chemical composition that eliminates the need of acidification of fermentable slurry during ethanol production.

Another object of the invention is to eliminate the use of sulfuric acid and therefore the accumulation of sulfur in grain co-products of ethanol production.

Another object of the invention is to prevent a negative effect on performance when animals are fed co-products produced using the present invention.

Another object of the invention is to eliminate the use of sulfuric acid in yeast used during sugarcane ethanol production therefore the accumulation of sulfur in dry yeast sold for animal or human uses is below the dangerous threshold level.

Another object of the invention is to eliminate the need of acidification of a fermentable slurry, eliminate the use of sulfuric acid, prevent a negative effect on animal performance and eliminate the use of sulfuric acid in yeast in any ethanol process that produces ethanol from carbohydrates.

The above objectives are accomplished when the present invention is used as follows:
a) mixing a fermentation feedstock with a fermentation broth containing yeast and/or an enzyme,
b) treating said mixture by adding a composition to the fermentor containing:
   10 - 90 wt. % of an antimicrobial aldehyde, preferably selected from the group consisting of formaldehyde, para-formaldehyde, glutaraldehyde and mixtures thereof,
   1- 50 wt. % of a surfactant having an HLB from 4 to 18,
   0 - 20 wt. % of an antimicrobial terpene, or essential oils,
   1- 50 wt. % of organic acids selected from C₁ to C₂₄ fatty acids, their salts, glycerides and esters thereof, and
   1- 50 wt. % water;
      wherein the concentration of aldehyde in the fermentor is from about 0.25 to 3 kg/MT of fermentation feedstock, and
c) improving ethanol yield.

The above objectives are accomplished when the present invention is applied in several areas (steps) during the process of ethanol production, i.e. from harvesting through fermentation. The following are the areas where the present invention can be applied:
a) In the fermenters
b) Before cooling the cooked grain
c) When liquefying raw grain
d) When liquefying cooked grain
e) In the slurry tanks
f) After cooling cooked grain
g) After extraction of juice from sugarcane or sugar beets
h) When clarifying sugarcane or beets extracts
i) In recycled yeast
j) During yeast propagation

### DESCRIPTION OF THE PREFERRED EMBODIMENTS

### Definitions

"Weight percent" (wt.%) of a component is based on the total weight of the formulation or composition in which the component is included.

"Aldehyde" includes formaldehyde, paraformaldehyde, and other antimicrobial aldehydes.

"Organic acid" includes formic, acetic, propionic, butyric and other C₁ to C₂₄ fatty acids, or mono-, di-, or triglycerides of C₁ to C₂₄ organic fatty acids or their alkyl esters.

"Antimicrobial terpene" can include allyl disulfide, citral, pinene, nerol, geraniol, carvacrol, eugenol, carvone, anethole, camphor, menthol, limonene, farnesol, carotene, thymol, borneol, myrcene, terpenene, linalool, or mixtures thereof. More specifically, the terpenes may comprise allyl disulfide, thymol, citral, eugenol, limonene, carvacrol, and carvone, or mixtures thereof. The terpene component may include other terpenes with anti-microbial properties and essential oils.

Bacteria that may interfere with ethanol fermentation include Lactobacillus and Leuconostoc, which cause the most problems. Other such bacteria include Pediococcus, Staphylococcus, Streptococcus, Bacillus, Enterococcus, Acetobacter, Gluconobacter, Clostridia, *A.pasterurianus, B. Subtilis, Leuconostoc mesenteroides, Weissella paramesenteroides* and other bacteria which reduce fermentation efficiency.

In ethanol produced from corn, antibiotics are the common biocides used, e.g., virginiamycin, penicillin, clindamycin, tylosin, chloramphenicol, cephalosporin, monensin and tetracycline. However, because the end product (bagasse) is not fed to animals when ethanol is produced from sugarcane, other biocides can be used since residues do not present the same problem. In such cases suitable biocides include carbamates, quaternary ammonium compounds, phenols and antibiotics (e.g., virginiamycin, penicillin, clindamycin, tylosin, chloramphenicol, cephalosporin and tetracycline).

The term "effective amount" of a compound means an amount capable of performing the function or having the property for which the effective amount is expressed, such as a nontoxic but sufficient amount to provide anti-microbial benefits e.g. biocide, biofilm preventer disrupter. Thus an effective amount may be determined by one of ordinary skill in the art by routine experimentation.

Formulations vary not only in the concentrations of the major components, e.g., aldehydes and organic acids, but also in the type of terpenes, surfactant(s) and water concentration. This invention can be modified by adding or deleting the terpene, type of organic acid, and using other types of surfactant.

### Composition(s)

In general, a composition of the invention contains:
a) 10 - 90 wt.% of an aldehyde, preferably selected from the group consisting of formaldehyde, paraformaldehyde, glutaraldehyde and mixtures thereof,
b) 1- 50 wt.% of a surfactant having an HLB from 4 to 18,
c) 1- 20 wt.% of an antimicrobial terpene, or essential oils,
d) 1- 50 wt.% of an organic acid or mixtures of organic acids selected from acetic, propionic, butyric, or other C₁ to C₂₄ fatty acids, salt forms, glycerides and esters thereof, and,
e) 1- 50 wt% water.

The antimicrobial terpenes, plant extracts or essential oils containing terpenes can be used in the compositions of this invention as well as the more purified terpenes. Terpenes are readily available commercially or can be produced by methods known in the art, such as solvent extraction or steam extraction/distillation or chemical synthesis.

The surfactant is non-ionic including ethoxylated castor oil surfactants with 1 to 200 ethylene molecules distributed normally around the mean, preferably a mean of 10 to 80. Other surfactants with similar characteristics can be used including polysorbate surfactants.

### Methods

The mixture of the present invention is applied by a spray nozzle.

The mixture of the present invention is applied mixed with a soluble carrier to the fermentable carbohydrate.

The mixture of the present invention is applied mixed in a starch-based carrier to the fermentable carbohydrate.

The mixture of the present invention is mixed with a liquid or solid carrier prior to be added to the fermentable carbohydrate.

The mixture is applied drop-wise on the fermentable broth or slurry.

The mixture of the present invention is applied in an in-line injection system.

The mixture of the present invention is applied in any or all of the treatable areas during production of sugar and ethanol from sugarcane.

The mixture of the present invention is applied in any or all of the treatable areas during production of sugar and ethanol from sugar beet.

The mixture of the present invention is applied in any or all of the treatable areas during production of ethanol from corn.

The mixture of the present invention is applied in any or all of the treatable areas during production of ethanol from other starchy materials different than corn.

The mixture of the present invention is applied in any or all of the treatable areas during production of ethanol from a cellulosic material.

The mixture is applied so as to provide a uniform and homogeneous distribution throughout the carbohydrate substrate.

### EXAMPLE 1

This example shows the base formulation "A" product used in all the following examples.

| **Table 1. Components of Formulation "A"** | |
|---|---|
| **Ingredient** | **(%)** |
| Formalin (37%) | 90.00 |
| Propionic Acid | 9.00 |
| d-limonene (terpene) | 0.35 |
| Polysorbate 80 (surfactant) | 0.65 |

### EXAMPLE 2

The purpose of this experiment was to determine if the need to decrease pH with sulfuric acid can be replaced by treating the fermenting corn slurry with Formulation "A". The following fermentation protocol was used.

### Fermentation procedure:

1. 0.55 kg of 1mm ground corn was added to 2.75 lt. of water. After mixing, 220ul of alpha-amylase was added to the slurry and mixed.
2. The mixture was cooked at 185-190°F for 2.5 hours.
3. After cooking the mixture was cooled down to 90°F
4. The cooked slurry was divided into two equal portions, referred as X and Y
5. The pH of portion X was adjusted to pH 4.2 using 10N sulfuric acid.
6. The pH of portion Y was kept as prepared.
7. To each portion, 2.5 gr dry yeast/1500 ml, 1.0 ml glucoamylase/1500 ml and 2.5 gr urea/1500 ml slurry were added and mixed.
8. 150 ml of cool slurry X was added to 9 fermenters (3 repetitions/treatment level).
9. 150 ml of cool slurry Y was added to 9 fermenters (3 repetitions/treatment level).
10. Formulation "A" was added as described in Table 2.
11. All fermenters were placed in a water bath at 110°F for 50 hours. All fermenters were kept stirring during fermentation.

| **Table 2. Formulation "A" treatments** | | | |
|---|---|---|---|
| **TRT** | **Treatment** | **Formulation "A" (ml)** | **#of Fermenters** |
| 1 | Control - pH 4.2 | 0.000 | 3 |
| 2 | Control - pH 5.9 | 0.000 | 3 |
| 3 | Formulation "A"- pH 4.2 at 0.5 kg/MT | 0.015 | 3 |
| 4 | Formulation "A"- pH 5.9 at 0.5 kg/MT | 0.015 | 3 |
| 5 | Formulation "A"- pH 4.2 at 1.0 kg/MT | 0.030 | 3 |
| 6 | Formulation "A"- pH 5.9 at 1.0 kg/MT | 0.030 | 3 |

### Chemical Measurements:

**Ethanol:** Ethanol was determined using a DIET-500 ethanol assay from QuantiChrom.
**Glucose:** Glucose was determined using a DIGL-100 glucose assay kit from QuantiChrom .

**Dry matter:** Dry matter was gravimetrically determined by sampling 10 gr. mixture from each fermenter and drying the mixture at 130°C for 2 hours.

| **Table 3. Chemical Results - Example #2** | | | | | |
|---|---|---|---|---|---|
| **TRT** | **pH** | **Formulation "A"** | **dry matter (%)** | **glucose g/dl** | **ethanol w/w** |
| Control | 4.2 | 0 kg/MT | 16.23 | 1.88 | 0.61 |
| Control | 5.9 | 0 kg/MT | 15.97 | 1.87 | 0.68 |
| Formulation "A" | 4.2 | 0.5 kg/MT | 16.32 | 1.82 | 0.44 |
| Formulation "A" | 5.9 | 0.5 kg/MT | 15.70 | 2.02 | 0.51 |
| Formulation "A" | 4.2 | 1.0 kg/MT | 16.09 | 2.32 | 0.44 |
| Formulation "A" | 5.9 | 1.0 kg/MT | 15.62 | 1.99 | 0.68 |

In a minimal contamination environment (< 100 cfu Lactobacillus/ml) as in this example, the optimum pH for fermentation was 5.9 as compared to pH 4.2 based on ethanol yield.

### EXAMPLE 3

The purpose of this experiment was to determine if the need to decrease pH with sulfuric acid can be replaced by treating the fermenting corn slurry with Formulation "A" added after cooking of the starch. The following fermentation protocol was used. In this example the corn slurry was contaminated with a Lactobacillus culture in order to simulated field conditions where the presence of higher levels of Lactobacillus is detrimental for optimum ethanol yield.

### Fermentation procedure:

1. 0.55 kg of 1mm ground corn was added to 2.75 lt. of water. After mixing, 220ul of alpha-amylase to the slurry was added and mixed.
2. The mixture was cooked at 185-190°F for 2.5 hours.
3. After cooking the mixture was cooled down to 90°F.
4. The cooked slurry was divided into two equal portions, referred as X and Y.
5. The pH of portion X was adjusted to pH 4.2 using 10N sulfuric acid.
6. The pH of portion Y was kept as prepared.
7. To each portion 2.5 gr dry yeast/1500 ml, 1.0 ml glucoamylase/1500 and 2.5 gr urea/1500 ml slurry was added and mixed.
8. Then 1000 ul of an overnight Lactobacillus culture grown in MRS broth(de Man, Rogosa and Sharpe broth) was added (approx. 10⁹ cfu/ml) to each 1500 ml slurry.
9. After mixing, 150 ml of cool slurry X was added to 9 fermenters (3 repetitions/treatment level).
10. 150 ml of cool slurry Y was added to 9 fermenters (3 repetitions/treatment level).
11. Formulation "A" was added as described in table 2.
12. All fermenters were placed in a water bath at 110°F for 50 hours. All fermenters were kept stirring during fermentation.

### Chemical Measurements:

**Ethanol:** Ethanol was determined using a DIET-500 ethanol assay from QuantiChrom.
**Glucose:** Glucose was determined using a DIGL-100 glucose assay kit from QuantiChrom .
**Dry matter:** Dry matter was gravimetrically determined by sampling 10 gr. mixture from each fermenter and drying the mixture at 130°C for 2 hours.

| **Table 4. Chemical results from Example #3** | | | | | |
|---|---|---|---|---|---|
| **TRT** | **pH** | **Formulation "A"** | **dry matter (%)** | **glucose (g/dl)** | **ethanol (w/w)** |
| Control | 4.2 | 0 kg/MT | 16.60 | 10.83 | 0.467 |
| Control | 5.9 | 0 kg/MT | 15.60 | 12.02 | 0.460 |
| Formulation "A" | 4.2 | 0.5 kg/MT | 16.80 | 13.97 | 0.383 |
| Formulation "A" | 5.9 | 0.5 kg/MT | 15.78 | 12.36 | 0.477 |
| Formulation "A" | 4.2 | 1.0 kg/MT | 16.57 | 13.17 | 0.429 |
| Formulation "A" | 5.9 | 1.0 kg/MT | 15.97 | 12.17 | 0.490 |

Higher ethanol yield was obtained when Formulation "A" was used in the fermenters. It is not necessary to decrease the pH to obtain maximum ethanol yield if Formulation "A" is used.

### EXAMPLE 4

In this example a variation of the fermentation protocol was used. An incubation step of 4 hours was added prior to the addition of the yeast. Yeast and Lactobacillus levels after fermentation were determined in this example. The new protocol is described below:

### Fermentation procedure:

1. 0.55 kg of 1mm ground corn was added to 2.75 lt. of water. After mixing, 220ul of alpha-amylase to the slurry was added and mixed.
2. The mixture was cooked at 185-190°F for 2.5 hours.
3. After cooking the mixture was cooled down to 90°F.
4. The cooked slurry was divided into two equal portions, referred as X and Y.
5. The pH of portion X was adjusted to pH 4.2 using 10N sulfuric acid.
6. The pH of portion Y was kept as prepared.
7. After mixing 1.0 ml glucoamylase/1500 ml and 2.5 gr urea/1500 ml slurry were added to each portion.
8. Then 1000 ul of an overnight lactobacillus culture grown in MRS was added (approx. 10⁹ cfu/ml).
9. After mixing, 150 ml of cool slurry X was added to 9 fermenters (3 repetitions/treatment level).
10. 150 ml of cool slurry Y was added to 9 fermenters (3 repetitions/treatment level).
11. Formulation "A" was added as described in Table 2.
12. Fermenters were let to incubate at room temperature for 4 hours.
13. After incubation, to each fermenter, 0.25 gr. dry yeast /fermenter was added.
14. All fermenters were placed in a water bath at 90°F for 50 hours. All fermenters were kept stirring during fermentation.

### Microbiological analysis:

**Lactobacillus:** After fermentation, triplicate samples/fermenter were taken and plated to determine Lactobacillus levels on MRS broth containing 1.5% Difco™ Agar. Plates were incubated in an anaerobic chamber at 37°C for 48 hours and colonies were enumerated.
**Yeast:** After fermentation, triplicate samples/fermenter were taken and plated on PDA for the determination of yeast levels. Plates were incubated at 27°C for 48 hours and colonies were enumerated.

| **Table 5. Chemical and Microbiological Results from Example #5** | | | | | | | |
|---|---|---|---|---|---|---|---|
| **TRT** | **pH** | **Formulation "A"** | **glucose (g/dl)** | **dry matter (%)** | **ethanol (w/w)** | **Lactobacillus (cfu/ml)** | **Yeast (cfu/ml)** |
| Control | 4.2 | 0 kg/MT | 0.02 | 5.46 | 2.29 | 5.9 x 10⁶ | 2.4 x 10⁷ |
| Control | 5.9 | 0 kg/MT | 0.06 | 5.12 | 2.51 | 3.6 x 10⁷ | 1.0 x 10⁷ |
| Formulation "A" | 4.2 | 0.5 kg/MT | 0.02 | 5.22 | 2.63 | 5.4 x 10⁵ | 4.2 x 10⁷ |
| Formulation "A" | 5.9 | 0.5 kg/MT | 0.01 | 4.92 | 3.08 | 2.0 x 10⁷ | 1.8 x 10⁷ |
| Formulation "A" | 4.2 | 1.0 kg/MT | 0.16 | 6.67 | 2.05 | 4.4 x 10⁴ | 1.1 x 10⁷ |
| Formulation "A" | 5.9 | 1.0 kg/MT | 0.01 | 4.33 | 2.59 | 4.2 x 10⁶ | 5.2 x 10⁷ |

Better ethanol yield was obtained when pH was un-adjusted and a low level of Formulation "A" was used. The low pH decreased Lactobacillus counts and the addition of formulation "A" decreased it further. There was no negative effect of formulation "A" on yeast counts.

### Example 5

This example is a repetition of example 5.

| **Table 6. Chemical and Microbiological Results from Example #6** | | | | | | | |
|---|---|---|---|---|---|---|---|
| **TRT** | **pH** | **Formulation "A"** | **glucose (g/dl)** | **dry matter (%)** | **ethanol (w/w)** | **Lactobacillus (cfu/ml)** | **Yeast (cfu/ml)** |
| Control | 4.2 | 0 kg/MT | 0.10 | 4.92 | 2.41 | 6.0 x 10⁵ | 6.2 x 10⁷ |
| Control | 5.9 | 0 kg/MT | 0.10 | 4.64 | 2.73 | 3.7 x 10⁵ | 1.7 x 10⁷ |
| Formulation "A" | 4.2 | 0.5 kg/MT | 0.18 | 4.67 | 2.90 | 3.3 x 10⁴ | 1.7 x 10⁷ |
| Formulation "A" | 5.9 | 0.5 kg/MT | 0.24 | 4.67 | 2.80 | 1.9 x 10⁸ | 6.2 x 10⁷ |
| Formulation "A" | 4.2 | 1.0 kg/MT | 0.18 | 4.79 | 2.77 | 2.0 x 10⁵ | 4.1 x 10⁷ |
| Formulation "A" | 5.9 | 1.0 kg/MT | 0.25 | 4.72 | 2.88 | 4.1 x 10⁷ | 4.0 x 10⁷ |

It will be apparent to those skilled in the art that variations and modifications of the invention can be made without departing from the spirit and scope of the teachings above. It is intended that the specification and examples be considered as exemplary only and are not restrictive.

## Claims

1. An improved method of ethanol fermentation with decreased use of acidifiers, comprising:
a) mixing a fermentation feedstock with a fermentation broth containing yeast and/or an enzyme,
b) treating said mixture by adding a composition to the fermentor containing:
10 - 90 wt. % of an antimicrobial aldehyde,
1- 50 wt. % of a surfactant having an HLB from 4 to 18,
0 - 20 wt. % of an antimicrobial terpene, or essential oils,
1- 50 wt. % of organic acids selected from C₁ to C₂₄ fatty acids, their salts, glycerides and esters thereof, and
1- 50 wt. % water;
wherein the concentration of aldehyde in the fermentor is from about 0.25 to 3 kg/MT of fermentation feedstock, and
c) isolating ethanol and improving yield.

2. The method of claim 1, wherein the aldehyde is selected from the group consisting of formaldehyde, para-formaldehyde, glutaraldehyde, and mixtures thereof.

3. The method of any one of the preceding claims, wherein the fermentation feedstock is corn, sorghum, wheat, triticale, rye, barley, rice or tubers.

4. The method of any one of the preceding claims, wherein the fermentation feedstock is sugarcane or sugar beet.

5. The method of any one of the preceding claims, wherein the carbohydrate to be fermented is derived from cellulose.

6. The method of any one of the preceding claims, which is free of antimicrobial agents or sulfuric acid.

7. A method to produce ethanol co-products with low sulfur content comprising the use of method of any one of the preceding claims.

8. A method to produce a low sulfur dry yeast resulting from sugarcane or sugar beet fermentation comprising the use of method of any one of the preceding claims.

9. The method of any one of the preceding claims, wherein the aldehyde is formaldehyde or para-formaldehyde.
